# EUROPÄISCHE PATENTSCHRIFT

(11) **EP 2 998 723 B1**
(45) Veröffentlichungstag und Bekanntmachung des Hinweises auf die Patenterteilung: **20.12.2017**
(21) Anmeldenummer: 15183492.6
(22) Anmeldetag: 02.09.2015
(51) Int. Cl.: G01N 9/00, A24C 5/14, A24C 5/34

(54) **MIKROWELLENSTRANGMESSVORRICHTUNG, VERFAHREN UND VERWENDUNG**
MICROWAVE STRAND MEASURING DEVICE, METHOD AND USE
DISPOSITIF DE MESURE A MICRO-ONDES D'UNE BARRE, PROCEDE ET UTILISATION

(30) Priorität: 18.09.2014 DE 102014218814
(43) Veröffentlichungstag der Anmeldung: 23.03.2016
(73) Patentinhaber: Hauni Maschinenbau GmbH, 21033 Hamburg (DE)
(72) Erfinder: MÜLLER, Johannes, 22767 Hamburg (DE); LEIFELS, Tobias, 20535 Hamburg (DE)
(74) Vertreter: Seemann & Partner Patentanwälte mbB

(56) Entgegenhaltungen:
- EP-A1- 1 855 104
- WO-A1-00/77501
- DE-A1-102011 083 051
- DE-B4-102004 017 597
- EBBE NYFORS AND PERTTI VAINIKAINEN: "INDUSTRIAL MICROWAVE SENSORS", INDUSTRIAL MICROWAVE SENSORS, XX, XX, 1. Januar 1989 (1989-01-01), XP002334964,

## Beschreibung

Die Erfindung betrifft eine Mikrowellenstrangmessvorrichtung, insbesondere zur Messung von Eigenschaften eines Materialstrangs der Tabak verarbeitenden Industrie, mit einem Mikrowellenresonatorgehäuse mit wenigstens einem als Mikrowellenresonator ausgebildeten im Wesentlichen hohlzylindrischen oder quaderförmigen Innenraum, der durch einen Materialstrang durchsetzbar ist, wobei Endflächen des Mikrowellenresonators in der Symmetrieachse des Mikrowellenresonators zentrale Durchführungsöffnungen für einen Materialstrang aufweisen, mit zwei Antennen, die zum Ein- und Auskoppeln eines Mikrowellenmessfelds in den Mikrowellenresonator im Mikrowellenresonator angeordnet sind. Die Erfindung betrifft ferner ein Verfahren zum Betreiben einer entsprechenden Mikrowellenstrangmessvorrichtung und eine Verwendung.

Die Erfindung betrifft insbesondere das Gebiet der Strangbildung und Strangverarbeitung in der Tabak verarbeitenden Industrie, also die Erzeugung von Zigarettensträngen und Filtersträngen in Strangmaschinen. Beispielsweise wird ein Zigarettenstrang erzeugt, indem zunächst Tabak auf einen Strangförderer aufgeschauert wird, der Tabakstrang mit einem Umhüllungspapierstreifen umhüllt wird und anschließend von dem Tabakstrang Zigaretten mehrfacher Gebrauchslänge abgelängt werden. Das Bilden des Tabak- oder Filterstrangs und das anschließende Schneiden bzw. Ablängen des Strangs erfolgt mit hoher Geschwindigkeit. Typisch sind bei heutigen Zigaretten- und Filterherstellungsmaschinen Stranggeschwindigkeiten von 10 m/s, wobei bei 100 mm Abschnittslänge ein Schnitttakt von 100 pro Sekunde folgt.

Die Qualität der Zigaretten hängt vom Zustand des Tabaks im Zigarettenstrang ab. Aus diesem Grund ist vorgesehen, die Feuchtigkeit und die Dichte des Tabaks im Zigarettenstrang zu messen und insbesondere die Dichte zu regeln. Weiterhin wird im Falle von plötzlichen und kurzzeitigen Signalschwankungen auf das Vorhandensein von Fremdkörpern geschlossen, wobei die entsprechenden Strangabschnitte nachfolgend ausgesondert werden. Eine entsprechende Überwachung erfolgt auch bei der Bildung von Filtersträngen.

Dies geschieht in modernen Zigarettenherstellungsmaschinen mit der Hilfe von Mikrowellenmesseinrichtungen, die wenigstens ein Mikrowellen-Resonatorgehäuse aufweisen, durch das der Tabakstrang hindurchgeführt wird.

Ein solches Resonatorgehäuse ist aus DE 198 54 550 B4 bekannt. Das dort gezeigte Resonatorgehäuse für Mikrowellen wird von einem Strang der Tabak verarbeitenden Industrie durchsetzt und besteht aus einem metallischen Material mit niedrigem Temperaturausdehnungskoeffizient. Dem Resonatorgehäuse sind zwecks Erfassung der Masse und/oder der Feuchte des Strangmaterials Mikrowellen zuführbar. Dieses Gehäuse weist eine Temperaturregelanordnung mit einem Fühler für die Temperatur des Gehäuses und einem Heiztransistor auf, wobei der Fühler den Heiztransistor derart steuert, dass dessen Verlustwärme die Temperatur des Gehäuses zumindest annähernd konstant über der Umgebungstemperatur hält.

Ferner ist aus DE 10 2011 083 051 A1 ein Mikrowellenresonatorgehäuse mit wenigstens einem als Mikrowellenresonator ausgebildeten im Wesentlichen hohlzylindrischen Innenraum, der durch einen Materialstrang der Tabak verarbeitenden Industrie durchsetzbar ist und dem Mikrowellen zuführbar sind, bekannt, wobei die Endflächen des Mikrowellenresonators in der Symmetrieachse des Mikrowellenresonators zentrale Durchführungsöffnungen für einen Materialstrang aufweisen, bei dem wenigstens eine der beiden Endflächen in einem ringförmigen Bereich um die Durchführungsöffnung herum in einen umlaufenden und radial nach innen überstehenden Kragen münden, dessen innere Öffnung eine Durchführungsöffnung für den Materialstrang bildet und der eine von dem den Mikrowellenresonator bildenden Innenraum abgewandte Rückseite aufweist, an der sich der Innendurchmesser des Gehäuses gegenüber dem Durchmesser der Durchführungsöffnung unter Bildung einer ringförmigen Hinterdrehung erweitert. Die Kragen mit den entsprechenden Hinterdrehungen sorgen dafür, dass die Eindringtiefe des Mikrowellenfeldes in den Materialstrang erhöht wird und dass die Erstreckung des Mikrowellenfeldes in Strangrichtung stark begrenzt wird. Damit wird sowohl die Ortsauflösung als auch die Sensitivität für die Strangmasse bzw. für Fremdkörper im Zentrum des Stranges erhöht.

Gegenüber diesem Stand der Technik liegt der vorliegenden Erfindung die Aufgabe zugrunde, eine Mikrowellenstrangmessvorrichtung, ein Verfahren zum Betreiben einer entsprechenden Mikrowellenstrangmessvorrichtung und eine Verwendung derselben zur Verfügung zu stellen, mit denen eine weiter verbesserte Messung von Eigenschaften eines Materialstrangs der Tabak verarbeitenden Industrie ermöglicht wird.

Diese Aufgabe wird gelöst durch eine Mikrowellenstrangmessvorrichtung, insbesondere zur Messung von Eigenschaften eines Materialstrangs der Tabak verarbeitenden Industrie, mit einem Mikrowellenresonatorgehäuse mit wenigstens einem als Mikrowellenresonator ausgebildeten im Wesentlichen hohlzylindrischen oder quaderförmigen Innenraum, der durch einen Materialstrang durchsetzbar ist, wobei Endflächen des Mikrowellenresonators in der Symmetrieachse des Mikrowellenresonators zentrale Durchführungsöffnungen für einen Materialstrang aufweisen, mit zwei Antennen, die zum Ein- und Auskoppeln eines Mikrowellenmessfelds in den Mikrowellenresonator im Mikrowellenresonator angeordnet sind, die dadurch weitergebildet ist, dass wenigstens eine Antenne den Mikrowellenresonator von einer Endfläche zur anderen Endfläche vollständig durchdringt und außerhalb des Mikrowellenresonators an einer Seite mit einem Mikrowellengenerator und/oder einer Messelektronik leitend verbunden oder verbindbar ist und an der gegenüberliegenden Seite mit einer Masse leitend verbunden oder verbindbar ist.

Die vorliegende Erfindung beruht auf dem Grundgedanken, dass durch die Verwendung von Antennen, die den zylindrischen oder quaderförmigen Mikrowellenresonator von einer Endfläche zur anderen Endfläche vollständig durchdringen und an einer Seite mit einer Masse leitend verbunden oder verbindbar sind, von einer bisher verfolgten vorwiegend kapazitiven Ein- und Auskopplung des Mikrowellenmessfeldes in den Mikrowellenresonator übergegangen wird zu einer induktiven Ein- und Auskopplung. Bei der erfindungsgemäßen induktiven Einkopplung der schwingungsfähigen Mode des Resonators wird diese in erster Linie über das Magnetfeld angeregt. Bei der elektrischen Einkopplung erfolgt dies über das elektrische Feld. Die induktive Einkopplung hat gegenüber der elektrischen Einkopplung die Vorteile, dass die anregenden Felder sauberer in das Feld des Resonators einkoppeln und weniger Feldverzerrungen auftreten. Dies verringert auch die parasitäre direkte Kopplung zwischen Ein- und Ausgang. Dies hat zur Folge, dass die Resonanzkurve des Mikrowellenresonators eine bessere Symmetrie aufweist und die externe Güte über die Position der Antenne bestimmt wird und nicht, wie bei der elektrischen Einkopplung, von der Länge der "Antennen" abhängt. Es ist daher kein Einstellen der Länge erforderlich.

Es lassen sich außerdem sehr einfach auch geringe externe Güten einstellen, die zu einer starken Kopplung führen. Bei der nicht erfindungsgemäßen elektrischen Einkopplung führt dies zu derart starken Feldverzerrungen, dass die eigentliche schwingende Mode des Resonators, insbesondere bei verlustbehafteter Beladung, kaum noch angeregt wird. Bei der induktiven Einkopplung hingegen ist die Resonanzkurve weiterhin messbar. Solche geringen externen Güten sind besonders vorteilhaft beim Messen von stark verlustbehafteten Materialien, beispielsweise bei hohen Beladungen von Aktivkohle.

Die Feldverzerrungen bei der elektrischen bzw., gleichbedeutend, kapazitiven Kopplung resultieren aus den hohen elektrischen Feldstärken an den Enden der langen, metallischen Stifte, die überlicherweise als Einkopplungsantennen und Auskopplungsantennen dienen. Die Stärke dieser Kopplung wird üblicherweise über die Länge der Stifte eingestellt. Die Kopplungsstärke steigt mit der Eindringtiefe der Stifte in den Resonator. Über diese Kopplung lässt sich auch die externe Güte des Resonators einstellen, wobei die Güte mit steigender Kopplungsstärke sinkt.

Derzeit wird die richtige Kopplungsstärke vor Inbetriebnahme eingestellt, indem die metallischen Stifte mit Einstellschrauben von der Gegenseite hineingedrückt werden. Diese Einstellschrauben sind in einem speziellen Resonatordeckel angebracht. In der Ausgangsposition müssen die Stifte tiefer in den Resonator eindringen, als in ihrer finalen Stellung, da ihre Eindringtiefen mit den Einstellschrauben ausschließlich verringert werden kann. Diese Art der Einstellung entfällt mit der erfindungsgemäßen magnetischen bzw., gleichbedeutend, induktiven Kopplung.

Bezogen auf die gewünschten Eigenwellen bzw. Schwingungsmoden des Resonators, die angeregt werden soll, ist dies bei einem zylindrischen Resonator die TM₀₁₀-Mode (auch E₀₁₀-Mode genannt). Diese hat die Maxima ihres elektrischen Felds im Zentrum des Resonators und das Maximum der magnetischen Feldstärke in der Nähe der Gehäusewand. Die Felder der als hineinragende Stifte ausgebildeten Antennen verkoppeln zwar mit der gewünschten Mode, jedoch auch in einem hohen Maße mit höheren Moden des Resonators. Diese höheren Moden sind unter Umständen bei der angeregten Frequenz nicht ausbreitungsfähig, und sie existieren nur in stark gedämpfter Form, sogenannte evaneszenten Moden. Die Anregung dieser höheren Moden führt zu einer direkten Verkopplung der Antennen. Diese unerwünschte Kopplung steigt mit der Frequenz an und ist der Grund für die charakteristische Asymmetrie der Resonanzkurve existierender Resonatoren. Beispielsweise bei der Verwendung von Resonanzfolgeverfahren kommt es dabei zu Berechnungsfehlern der Resonanzfrequenz, da die Approximation der Resonanzkurze einen symmetrischen Kurvenverlauf voraussetzt.

Die magnetische Einkopplung regt die höheren Moden in geringerem Maße an und führt zu einer verbesserten, verzerrungsfreien Einkopplung und weniger direkten Überkopplung vom Eingang auf den Ausgang. Dadurch ergibt sich ein annähernd symmetrischer Resonanzverlauf, so dass es bei dem Resonanzfolgeverfahren und anderen möglichen Verfahren weniger Fehler gibt und eine bessere Modellierung möglich ist.

Vorzugsweise durchdringen beide Antennen den Mikrowellenresonator von einer Endfläche zur anderen Endfläche vollständig. Damit ist gewährleistet, dass eine optimale induktive Ein- und Auskopplung des Mikrowellenfeldes in und aus dem Mikrowellenresonator erfolgt. Es ist aber im Rahmen der Erfindung auch vorgesehen, dass eine gemischte Ein- und Auskopplung erfolgt, beispielsweise eine induktive Einkopplung und eine wenigstens teilweise kapazitive Auskopplung oder umgekehrt.

In einer vorteilhaften Weiterbildung ist vorgesehen, dass die mit der Masse verbundene Seite oder Seiten der Antenne oder Antennen an einer Endfläche des Mikrowellenresonators mit dem Mikrowellenresonatorgehäuse leitend verbunden ist oder sind, so dass die Antenne oder die Antennen mit dem Mikrowellenresonatorgehäuse eine oder mehrere induktive Schleife oder Schleifen bildet oder bilden. Jede einzelne entsprechend ausgebildete Antenne bildet dabei eine induktive Schleife mit dem Mikrowellenresonatorgehäuse. Die zwischen dem Mikrowellengehäuse und der jeweiligen Antenne eingeschlossene Fläche wirkt sich auf die Einkopplungsstärke aus. Je größer diese Fläche ist, desto stärker ist die Einkopplung und desto geringer die Güte des Mikrowellenresonators.

Die Antennen sind vorzugsweise im Wesentlichen parallel zur Längsachse des Mikrowellenresonators angeordnet. Dies ermöglicht eine optimale Ankopplung an die TM₀₁₀-Mode im zylindrischen Resonator bzw. die entsprechenden Moden in quaderförmigen Resonatoren.

In einer vorteilhaften Weiterbildung von eigenständigen erfinderischen Rang ist vorgesehen, dass wenigstens eine Antenne, die den Mikrowellenresonator von einer Endfläche zur anderen Endfläche vollständig durchdringt, wenigstens abschnittsweise in ihrer axialen Symmetrie gebrochen ist und im Mikrowellenresonator drehbar gelagert angeordnet ist. Dadurch, dass eine in seiner axialen Symmetrie wenigstens abschnittsweise gebrochene Antenne drehbar gelagert ist, lässt sich durch eine Drehung dieser Antenne die Fläche der induktiven Schleife und somit die Stärke der Einkopplung bzw. Auskopplung und die Güte des Mikrowellenresonators einstellen. Bei der Brechung der axialen Symmetrie ist wenigstens ein Abschnitt der Antenne außerhalb der Drehachse gelagert, so dass sie sich bei einer Drehung der Antenne an die Seitenwand des Resonatorgehäuses annähert oder entfernt. Da die Feldvektoren des magnetischen Feldes bei der TM₀₁₀-Mode parallel zur zylindrischen Außenwand angeordnet sind, wird somit die effektive Fläche zwischen der äußeren Resonatorwand und der Antenne durch eine Drehung der Antenne, und somit die Einkopplungsstärke oder Auskopplungsstärke, verändert. Dies gilt entsprechend bei quaderförmigen Resonatoren.

Hierzu ist es vorteilhaft, wenn eine drehbar gelagerte Antenne an ihrem auf Masse verbundenen Ende ein Gewinde aufweist, mit dem sie in die einen Deckel des Mikrowellenresonatorgehäuses bildende Endfläche eindrehbar ist, wobei insbesondere auf einer masseseitigen Endfläche der Antenne ein Drehelement, insbesondere ein Schraubendreher-Schlitz oder Kreuzschlitz und/oder eine Rändelschraube, angeordnet ist. Das Drehelement bzw. der Schlitz können dann dazu benutzt werden, die Einkopplungsstärke oder Auskopplungsstärke und gegebenenfalls die Güte des Resonators einzustellen.

In der bevorzugten Weiterbildung ist eine drehbar gelagerte Antenne an ihrem der Masse gegenüberliegenden Ende durch einen dielektrischen Übergang in der einen Boden des Mikrowellenresonators bildenden Endfläche gesteckt oder steckbar und weist insbesondere einen Konnektor auf, der mit einer passenden Steckverbindung verbindbar ist.

Vorzugsweise sind die Antennen im Mikrowellenresonator so angeordnet, dass sie in das Feld der TM₀₁₀-Mode des Mikrowellenresonators möglichst verzerrungsfrei einkoppeln.

Die der Erfindung zugrundeliegende Aufgabe wird auch durch ein Verfahren zum Betreiben einer entsprechenden zuvor beschriebenen erfindungsgemäßen Mikrowellenstrangmessvorrichtung gelöst, bei dem eine Güte des Mikrowellenresonators mittels Drehung einer drehbar gelagerten Antenne im Mikrowellenresonator eingestellt wird, die den Mikrowellenresonator von einer Endfläche zur anderen Endfläche vollständig durchdringt und wenigstens abschnittsweise in ihrer axialen Symmetrie gebrochen ist.

Dieses Verfahren bezieht sich auf die zuvor beschriebene Mikrowellenstrangmessvorrichtung mit wenigstens einer drehbar gelagerten in ihrer axialen Symmetrie abstandsweise gebrochenen Antenne und weist damit die gleichen Vorteile, Merkmale und Eigenschaften auf wie die erfindungsgemäße Mikrowellenstrangmessvorrichtung hinsichtlich ihrer Einstellbarkeit und Abstimmbarkeit.

Das Verfahren wird vorteilhaft weitergebildet, indem die Drehung erfolgt, während die Antenne mit einem Mikrowellengenerator oder einer Messelektronik verbunden ist.

Die der Erfindung zugrundeliegende Aufgabe wird schließlich ebenfalls gelöst durch eine Verwendung wenigstens einer erfindungsgemäß zuvor beschriebenen Mikrowellenstrangmessvorrichtung zur Erkennung von Eigenschaften eines Strangs der Tabak verarbeitenden Industrie, insbesondere eines Tabakstrangs oder eines Filterstrangs, insbesondere einer Strangdichte, einer Feuchtigkeit, einer Beladung mit Zusatzstoffen, insbesondere mit Weichmacher oder Aktivkohle, zur Fremdkörpererkennung und/oder zur Erkennung von in einen Strang eingelegten Objekten, insbesondere flüssigkeitsgefüllten Kapseln.

Auch diese Verwendung ergibt die gleichen Vorteile, Eigenschaften und Merkmale wie das erfindungsgemäße Verfahren und die erfindungsgemäße Mikrowellenstrangmessvorrichtung.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines bekannten Resonatorgehäuses gemäß DE 10 2004 017 597 B4,
- Fig. 2: eine schematische Darstellung eines bekannten Resonatorgehäuses gemäß DE 10 2011 083 051 A1,
- Fig. 3: eine schematische Querschnittsdarstellung durch ein erfindungsgemäßes Resonatorgehäuse und
- Fig. 4a) - 4d): schematische Skizzen alternativer erfindungsgemäßer Antennenkonfigurationen.

In den Zeichnungen sind jeweils gleiche oder gleichartige Elemente und/oder Teile mit denselben Bezugsziffern versehen, so dass von einer erneuten Vorstellung jeweils abgesehen wird.

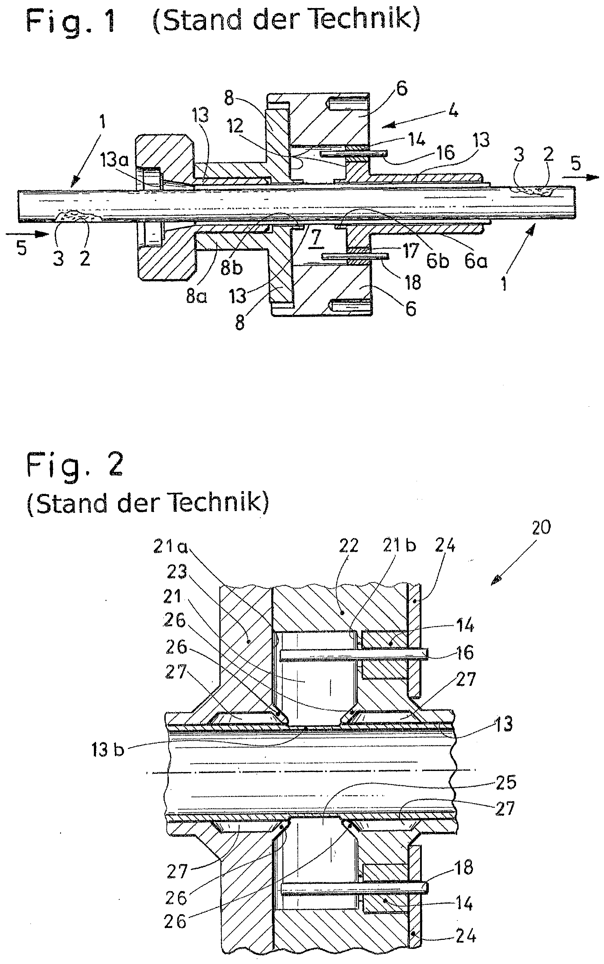

Fig. 1 zeigt einen teilweise aufgebrochenen, in Richtung des Pfeils 5 bewegten Materialstrang 1, in diesem Fall einen Zigarettenstrang, bestehend aus einem Füller 2 aus Schnitttabak und einer Umhüllung 3 aus Zigarettenpapier, der ein Resonatorgehäuse 4 durchsetzt, dem Mikrowellen zwecks Erfassung wenigstens einer Eigenschaft des Füllers 2, beispielsweise der Masse oder der Feuchte, zugeführt werden.

Das Resonatorgehäuse 4 gemäß DE 10 2004 017 597 B4 weist einen Hohlkörper in Form eines Hohlzylinders 6 auf, dessen Innenraum (Resonatorraum) 7 symmetrisch zu dem Zigarettenstrang 1 angeordnet ist. An ihm ist ein Deckel 8 zum Verschließen angeschraubt. Sowohl Hohlzylinder 6 als auch Deckel 8 bestehen aus nichtmetallischem Material mit einem sehr niedrigen Temperaturausdehnungskoeffizienten, beispielsweise einem Glas oder einer Glaskeramik. Wenigstens der Hohlzylinder 6 sollte aus dem nichtmetallischen Material mit dem sehr niedrigen Temperaturausdehnungskoeffizienten bestehen. In Folge der guten Konstanz der Geometrie des Resonatorgehäuses 4 und des Resonatorraums 7 lässt sich auch eine gute Konstanz der Messergebnisse erreichen.

Der Resonatorraum 7 des Resonatorgehäuses 4 ist mit einer dünnen Goldschicht 12 bedampft, die eine die Messwertkonstanz beeinträchtigende Korrosionsbildung zuverlässig verhindert.

Zum mechanischen Abschluss des Resonatorraums 7 gegenüber dem Zigarettenstrang 1 und von diesem eventuell angeförderten Schmutzteilchen, also zwecks Verhinderung einer Verschmutzung des Resonatorraums 7, die das Messergebnis beeinträchtigen würde, dient ein Schutzrohr 13, das vorteilhaft aus einer Substanz der Polyaryletherketon (PAEK)-Gruppe, z. B. aus Polyetheretherketon (PEEK) besteht. An einem seiner Enden 13a, an dem der Strang 1 in das Resonatorgehäuse 6 einläuft, ist das Schutzrohr 13 trichterförmig aufgeweitet.

Das Resonatorgehäuse 4 erstreckt sich außerhalb des Resonatorraums 7 rohrförmig (6a, 8a) auf beiden Seiten in Richtung des Strangs 1 nach außen, um den Austritt von Mikrowellen aus der Resonatorkammer zu verhindern. Es erstreckt sich auch rohrförmig (6b, 8b) etwas nach innen. Zur Einkopplung der von einem Mikrowellengenerator erzeugten Mikrowellen dient eine durch einen Isolierring 14 vom Hohlzylinder 6 isolierte Antenne 16. Zum Auskoppeln von Mikrowellen, die einer nicht dargestellten Auswertschaltung zugeführt werden sollen, dient eine durch eine Isolierung 17 vom Hohlzylinder 6 isolierte Antenne 18. Auf die Isolierungen 14 und 17 kann verzichtet werden, falls der Hohlzylinder 6 selbst nicht leitend ist. Bei einem leitfähigen nichtmetallischen Material des Hohlzylinders 6 sind die Isolierringe bzw. Isolierungen 14 und 17 für die Koppelantennen 16 und 18 notwendig.

Die rohrförmige Erstreckung 6b, 8b der Endflächen des Resonators 7 führen dazu, dass der Resonatorspalt gegenüber der Höhe des Resonators 7 verkleinert ist. Damit wird die Ortsauflösung verbessert, allerdings vermindert sich auch die Eindringtiefe des Mikrowellenfeldes in den Materialstrang 1 hinein. Zusätzlich ergibt sich eine relativ starke Ausdehnung des Mikrowellenfeldes in Strangrichtung außerhalb des Mikrowellenresonators 7, da das Gehäuse den Materialstrang 1 stromaufwärts und stromabwärts des Mikrowellenresonators 7 mit zylindrischen Innenflächen flach umgibt, so dass sich keine nennenswerte Bündelung am Ort des Mikrowellenresonators 7 und des Resonatorspaltes ergibt.

In Fig. 2 ist ein Ausschnitt aus einem Resonatorgehäuse 20 gemäß DE 10 2011 083 051 A1 dargestellt. Auch dieses Resonatorgehäuse 20 umgibt einen im Wesentlichen hohlzylindrischen Mikrowellenresonator 21 mit zwei flachen Endflächen 21a, 21b. Zwischen den Endflächen 21a, 21b befindet sich ein Resonatorspalt 25. Wenn, wie in Fig. 1 gezeigt, ein Materialstrang von links nach rechts durch den Mikrowellenresonator 21 geführt wird, ist die Endfläche 21a stromaufwärts und die Endfläche 21b stromabwärts angeordnet. Der Mikrowellenresonator 21 wird durch einen Hohlzylinder 22 begrenzt, der durch einen Deckel 23 stromaufwärts abgeschlossen ist. Stromabwärts schließt sich eine Leiterplatte 24 an.

Der Resonatorspalt 25 ist durch die Kragen 26, die von den Endflächen 21a, 21b nach innen abzweigen, begrenzt. Hierbei handelt es sich um umlaufende flächige ringförmige Körper mit schrägem, geradem Querschnitt. Hierdurch ist in einem zentrumsnahen ringförmigen Abschnitt die Höhe des Resonators 21 zwischen den Endflächen 21a, 21b verringert bis zum Resonatorspalt 25. An dieser Stelle stoßen die Kanten der Kragen 26 an das Schutzrohr 13, das in diesem Abschnitt 13b in seiner Wandstärke verringert ist.

Auf der jeweiligen Rückseite der Kragen 26 stromaufwärts bzw. stromabwärts des Mikrowellenresonators 21 sind im Hohlzylinder 22 bzw. dem Deckel 23 Hinterdrehungen 27 angeordnet, deren Grundflächen in radialer Richtung gegenüber den Kanten der Kragen 26 zurückgesetzt sind, also einen größeren Durchmesser aufweisen. Die Zurücksetzung der Grundfläche der Hinterdrehung 27 zusammen mit der schräg gestellten Hinterwand der jeweiligen Kragen 26 sorgen dafür, dass die Feldlinien des Mikrowellenfeldes sich auf dem Kragen 26 stark bündeln, so dass dieser gegenüber der Grundfläche der Hinterdrehung 27 wie eine Antenne wirkt. Diese Bündelung verhindert das Ausbreiten des Mikrowellenfeldes in die Durchführungsöffnung und in Längsrichtung des Materialstrangs. Gleichzeitig projiziert die Anordnung der Kragen 26 die Feldlinien des Mikrowellenfeldes in den Materialstrang hinein.

Die Hinterdrehungen 27 sind auf den vom Mikrowellenresonator 21 abgewandten Seiten wiederum abgeschlossen, so dass das Schutzrohr 13 an diesen Stellen gestützt wird und ein Austreten von Mikrowellen aus dem Resonatorgehäuse 20 durch die Hinterdrehungen 27 verhindert wird.

Die Anordnung und Wahl der Form der Kragen 26 hat den weiteren Effekt, dass Resonanzen im Mikrowellenresonator, die nicht der E₀₁₀-Mode entsprechen, verringert werden, so dass die dadurch auftretenden Messungenauigkeiten ebenfalls vermindert werden. Der dargestellte Fall zeigt die Kragen 26 stromaufwärts und stromabwärts des Mikrowellenresonators 21 in spiegelbildlicher Anordnung. Die Winkel, die die Kragen 26 jeweils mit den Endflächen 21a, 21b einschließen, betragen ca. 135°. Es ist aber auch möglich, verschiedene Winkel bei der Endfläche 21a und der Endfläche 21b einzustellen.

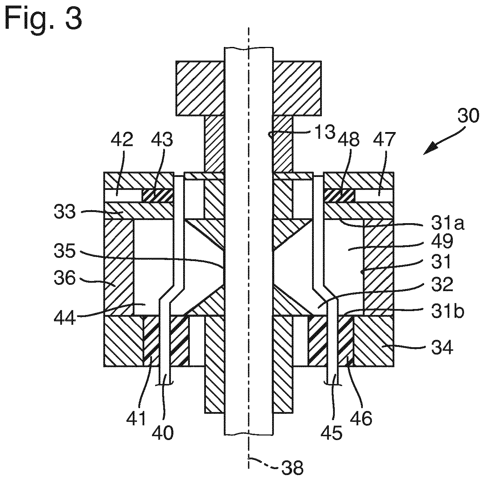

In Fig. 3 ist ein erfindungsgemäßes Mikrowellenresonatorgehäuse 30 mit einem Mikrowellenresonator 31 gezeigt, der mittels elektrisch leitfähiger Endflächen 31a, 31b und Deckel 33, Boden 34 und zylindrischer Seitenwand 36 zu einem Hohlzylinder 32 gebildet ist. Die Endfläche 31a befindet sich am Deckel 33, die Endfläche 31b am Boden 34. Das Mikrowellenresonatorgehäuse 30 ist geerdet und befindet sich elektrisch auf Masse.

Das Mikrowellenresonatorgehäuse 30 weist zentral konusförmige Abschnitte auf, so dass sich im zentralen Bereich ein Resonatorspalt 35 ergibt, der das Mikrowellenresonanzfeld bzw. Mikrowellenmessfeld auf einen kleinen Bereich im Zentrum bündelt. Ebenfalls dargestellt ist ein Schutzrohr 13, das zentral entlang einer Symmetrieachse 38 des Mikrowellenresonators 31 verläuft, und das zur Führung eines Materialstroms der Tabak verarbeitenden Industrie ausgebildet ist.

Der Mikrowellenresonator 31 verfügt über eine Einkopplungsantenne 40 und einer Auskopplungsantenne 45, die jeweils durch isolierende Durchführungen 41 bzw. 46 durch den Boden 34 in den Hohlzylinder 32 eindringen und bei dem Deckel 33 wieder austreten. Dort werden sie mittels Madenschrauben 43, 48, die in Gewindebohrungen 42, 47 im Deckel 33 angeordnet sind, festgeklemmt und elektrisch an diesem Ende jeweils ebenfalls auf Masse gelegt.

Die Antennen 40, 45 in Fig. 3 sind nicht vollständig linear ausgebildet. Sie sind an beiden Enden, d.h. im Deckel 33 und im Boden 34, fixiert.

Die Antennen 40, 45 koppeln induktiv an die TM₀₁₀-Mode an, wobei nur geringe Verzerrungen auftreten. Die Stärke der Einkopplung hängt von der umschlossenen Fläche 44 zwischen der Einkopplungsantenne 40 einerseits und dem äußeren umgebenden Rand des Mikrowellenresonators 31, d.h. dem äußeren Teil der Endflächen 31a, 31b bis zum Seitenwand 36 ab. Die Stärke der Auskopplung hängt wiederum von der umschlossenen Fläche 49 ab, die zwischen der Auskopplungsantenne 45 und den äußeren Teilen von Deckel 33, Boden 34 und Seitenwand 36 eingeschlossen ist.

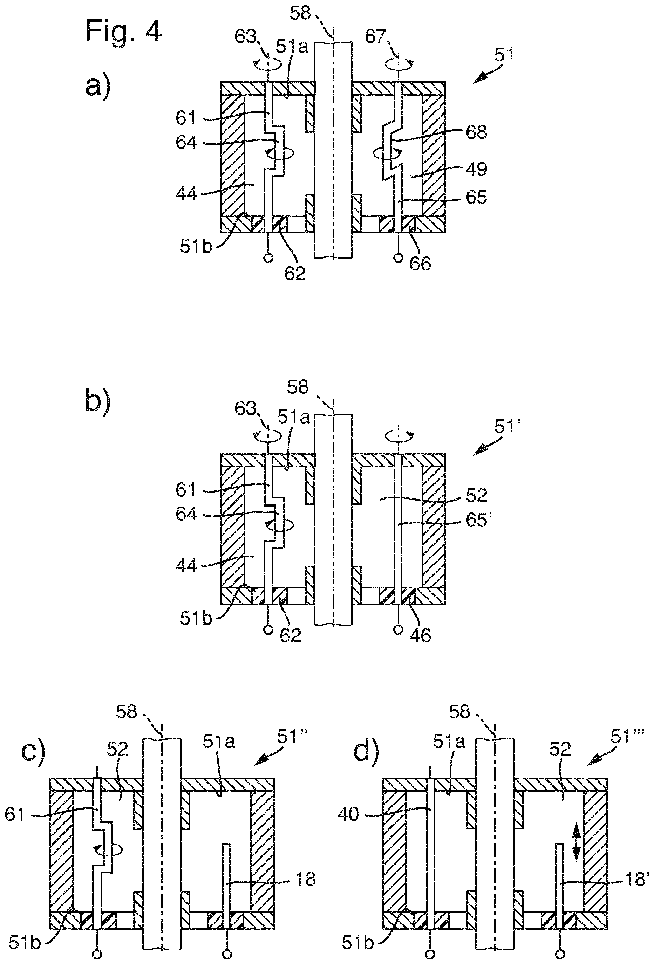

In Fig. 4a) bis 4d) sind vier verschiedene Beispiele erfindungsgemäßer und abstimmbarer Mikrowellenresonatoren 51 bis 51'" dargestellt. Fig. 4a) zeigt einen schematisch skizzierten Mikrowellenresonator 51 im Querschnitt, der als Einkopplungsantenne 61 und als Auskopplungsantenne 65 jeweils eine lineare Antenne mit ausgerücktem bzw. ausgestellten Abschnitt 64, 68 aufweist. Der ausgestellte Abschnitt 64, 68 befindet sich jeweils zentral auf der Höhe des Resonatorspalts 25. In beiden Fällen sind die Antennen 61, 65 drehbar auf einer Drehachse 63, 67 angeordnet, wobei eine elektrische leitende Verbindung zur Endfläche 51a und somit zur Masse vorhanden ist. In der Endfläche 51b ist jeweils eine isolierende Durchführung 62, 66 vorgesehen, die eine drehbare Lagerung enthält. Durch Drehung um die jeweilige Drehachse 63, 67, die zur Symmetrieachse 58 des Mikrowellenresonators 51 parallel ist, wird die umschlossene Fläche 44 bzw. 49 der induktiven Schleife, die aus Antenne 61 bzw. 65 und der äußeren Seitenwand des Hohlzylinders 52 des Mikrowellenresonators 51 gebildet wird, verändert und somit die Einkopplungsstärke, Auskopplungsstärke und Güte des Mikrowellenresonators abgestimmt.

In Fig. 4b) ist eine alternative Ausführungsform zu der Ausführungsform aus Fig. 4a) gezeigt. Während in Bezug auf die Einkopplungsantenne 61 die Anordnung die gleiche ist wie in Fig. 4a), ist bei dem Mikrowellenresonator 51' aus Fig. 4b) die Auskopplungsantenne 65' als lineare Antenne, die nicht drehbar ist, ausgebildet. In diesem Fall können nur die Einkopplungsstärke und die Güte des Mikrowellenresonators 51' eingestellt und abgestimmt werden.

In Fig. 4c) ist ein drittes Ausführungsbeispiel eines erfindungsgemäßen abstimmbaren Mikrowellenresonators 51" prinzipiell schematisch dargestellt, der sich von den Ausführungsbeispielen der Figuren 4a) und 4b) dadurch unterscheidet, dass die Auskopplungsantenne 18 nunmehr als Antenne für eine kapazitive, d.h. elektrische Auskopplung ausgebildet ist, die in den Hohlraum bzw. Hohlzylinder 52 des Mikrowellenresonators 51' hineinragt. Auch dieser Mikrowellenresonator 51" ist durch Drehung der Einkopplungsantenne 61 um ihre Drehachse abstimmbar.

Ein weiteres Ausführungsbeispiel ist in Fig. 4d) schematisch skizziert. In diesem Fall ist eine Einkopplungsantenne 40 als lineare nicht abstimmbare Antenne ausgebildet, die eine induktive Schleife bildet und somit eine induktive Einkopplung des Mikrowellenmessfeldes in den Mikrowellenresonator 51'" bewirkt. Eine Abstimmung erfolgt in Form der Länge der kapazitiven Auskopplungsantenne 18'. Diese ragt in den Hohlzylinder 52 der Mikrowellenmessvorrichtung 51'" hinein und kann zur Abstimmung in ihrer Eindringtiefe in den Hohlraum verändert werden.

Die Erfindung ist voranstehend am Beispiel eines im Wesentlichen zylindrischen bzw. hohlzylindrischen Mikrowellenresonators erläutert. Die Ausführungen gelten in analoger Weise auch für im Wesentlichen quaderförmige Resonatoren, wobei sich lediglich die geometrischen Gegebenheiten wie beispielsweise die angeregten Moden und die Frequenz oder Frequenzen des Mikrowellenmessfelds von denen in hohlzylindrischen Resonatoren unterscheiden können. Im Rahmen der Erfindung sind Merkmale, die mit "insbesondere" oder "vorzugsweise" gekennzeichnet sind, als fakultative Merkmale zu verstehen.

### Bezugszeichenliste

- 1: Materialstrang
- 2: Füller
- 3: Umhüllung
- 4: Resonatorgehäuse
- 5: Bewegungsrichtung
- 6: Hohlzylinder
- 6a: äußere Erstreckung Hohlzylinder
- 6b: innere Erstreckung Hohlzylinder
- 7: Resonatorraum
- 8: Deckel
- 8a: äußere Erstreckung Deckel
- 8b: innere Erstreckung Deckel
- 12: Goldschicht
- 13: Schutzrohr
- 13a: Schutzrohreinlass
- 13b: dünner Schutzrohrabschnitt
- 14: Isolierring
- 16: Einkopplungsantenne
- 17: Isolierung
- 18: Auskopplungsantenne
- 18': axial verschiebbare Auskopplungsantenne
- 20: Mikrowellenresonatorgehäuse
- 21: Mikrowellenresonator
- 21a, 21b: Endfläche
- 22: Hohlzylinder
- 23: Deckel
- 24: Leiterplatte
- 25: Resonatorspalt
- 26: Kragen
- 27: Hinterdrehung
- 30: Mikrowellenresonatorgehäuse
- 31: Mikrowellenresonator
- 31a, 31b: Endfläche
- 32: Hohlzylinder
- 33: Deckel
- 34: Boden
- 35: Resonatorspalt
- 36: zylindrische Seitenwand
- 38: Symmetrieachse
- 40: Einkopplungsantenne
- 41: isolierende Durchführung
- 42: Gewindebohrung
- 43: Madenschraube
- 44: umschlossene Fläche
- 45: Auskopplungsantenne
- 46: isolierende Durchführung
- 47: Gewindebohrung
- 48: Madenschraube
- 49: umschlossene Fläche
- 51 - 51'": Mikrowellenresonator
- 51a, 51b: Endfläche
- 52: Hohlzylinder
- 58: Symmetrieachse
- 61: Einkopplungsantenne
- 62: isolierende Durchführung
- 63: Drehachse
- 64: ausgestellter Abschnitt
- 65: Einkopplungsantenne
- 65': lineare Auskopplungsantenne
- 66: isolierende Durchführung
- 67: Drehachse
- 68: ausgestellter Abschnitt

## Patentansprüche

1. Mikrowellenstrangmessvorrichtung, insbesondere zur Messung von Eigenschaften eines Materialstrangs (1) der Tabak verarbeitenden Industrie, mit einem Mikrowellenresonatorgehäuse (30) mit wenigstens einem als Mikrowellenresonator (31, 51 - 51''') ausgebildeten hohlzylindrischen oder quaderförmigen Innenraum (32, 52), der durch einen Materialstrang (1) durchsetzbar ist, wobei Endflächen (31a, 31b, 51a, 51b) des Mikrowellenresonators (31, 51 - 51''') in der Symmetrieachse (38, 58) des Mikrowellenresonators (31, 51 - 51'") zentrale Durchführungsöffnungen für einen Materialstrang (1) aufweisen, mit zwei Antennen (18, 18', 40, 45, 61, 65, 65'), die zum Ein- und Auskoppeln eines Mikrowellenmessfelds in den Mikrowellenresonator (31, 51 - 51'") im Mikrowellenresonator (31, 51 - 51'") angeordnet sind, **dadurch gekennzeichnet, dass** wenigstens eine Antenne den Mikrowellenresonator (31, 51 - 51'") von einer Endfläche (31b, 51b) zur anderen Endfläche (31a, 51a) vollständig durchdringt und außerhalb des Mikrowellenresonators (31, 51 - 51''') an einer Seite mit einem Mikrowellengenerator und/oder einer Messelektronik leitend verbunden ist und an der gegenüberliegenden Seite mit einer Masse leitend verbunden ist.

2. Mikrowellenstrangmessvorrichtung nach Anspruch 1, **dadurch gekennzeichnet, dass** beide Antennen (40, 45, 61, 65, 65') den Mikrowellenresonator (31, 51 - 51'") von einer Endfläche (31b, 51b) zur anderen Endfläche (31a, 51a) vollständig durchdringen.

3. Mikrowellenstrangmessvorrichtung nach Anspruch 1 oder 2, **dadurch gekennzeichnet, dass** die mit der Masse verbundene Seite oder Seiten der Antenne (40, 45, 51, 65, 65') oder Antennen (40, 45, 61, 65, 65') an einer Endfläche (31a, 51a) des Mikrowellenresonators (31, 51 - 51'") mit dem Mikrowellenresonatorgehäuse (30) leitend verbunden ist oder sind, so dass die Antenne (40, 45, 51, 65, 65') oder die Antennen (40, 45, 51, 65, 65') mit dem Mikrowellenresonatorgehäuse (30) eine oder mehrere induktive Schleife oder Schleifen bildet oder bilden.

4. Mikrowellenstrangmessvorrichtung nach einem der Ansprüche 1 bis 3, **dadurch gekennzeichnet, dass** die Antennen (18, 18', 40, 45, 61, 65, 65') im Wesentlichen parallel zur Längsachse des Mikrowellenresonators (31, 51 - 51'") angeordnet sind.

5. Mikrowellenstrangmessvorrichtung nach einem der Ansprüche 1 bis 4, **dadurch gekennzeichnet, dass** wenigstens eine Antenne (61, 65), die den Mikrowellenresonator (51 - 51'") von einer Endfläche zur anderen Endfläche vollständig durchdringt, wenigstens abschnittsweise in ihrer axialen Symmetrie gebrochen ist und im Mikrowellenresonator (51 - 51''') drehbar gelagert angeordnet ist.

6. Mikrowellenstrangmessvorrichtung nach Anspruch 5, **dadurch gekennzeichnet, dass** eine drehbar gelagerte Antenne (61, 65) an ihrem auf Masse verbundenen Ende ein Gewinde aufweist, mit dem sie in die einen Deckel (33) des Mikrowellenresonatorgehäuses (30) bildende Endfläche (31a, 51a) eindrehbar ist, wobei insbesondere auf einer masseseitigen Endfläche der Antenne (61, 65) ein Drehelement angeordnet ist.

7. Mikrowellenstrangmessvorrichtung nach Anspruch 5 oder 6, **dadurch gekennzeichnet, dass** eine drehbar gelagerte Antenne (61, 65) an ihrem der Masse gegenüberliegenden Ende durch einen dielektrischen Übergang (41, 46, 62, 66) in der einen Boden (34) des Mikrowellenresonators (31, 51 - 51'") bildenden Endfläche (31b, 51b) gesteckt ist und einen Konnektor aufweist, der mit einer passenden Steckverbindung verbindbar ist.

8. Mikrowellenstrangmessvorrichtung nach Anspruch 1 bis 7, **dadurch gekennzeichnet, dass** die Antennen (40, 45, 61, 65, 65') im Mikrowellenresonator (31, 51 - 51'") so angeordnet sind, dass sie in das Feld der TM₀₁₀-Mode des Mikrowellenresonators (31, 51 - 51'") möglichst verzerrungsfrei einkoppeln.

9. Verfahren zum Betreiben einer Mikrowellenstrangmessvorrichtung nach einem der Ansprüche 5 bis 8, **dadurch gekennzeichnet, dass** eine Güte des Mikrowellenresonators (31, 51 - 51'") mittels Drehung einer drehbar gelagerten Antenne (61, 65) im Mikrowellenresonator eingestellt wird, die den Mikrowellenresonator (31, 51 - 51''') von einer Endfläche (31b, 51b) zur anderen Endfläche (31a, 51a) vollständig durchdringt und wenigstens abschnittsweise in ihrer axialen Symmetrie gebrochen ist.

10. Verfahren nach Anspruch 9, **dadurch gekennzeichnet, dass** die Drehung erfolgt, während die Antenne (61, 65) mit einem Mikrowellengenerator oder einer Messelektronik verbunden ist.

11. Verwendung wenigstens einer Mikrowellenstrangmessvorrichtung nach einem der Ansprüche 1 bis 8 zur Erkennung von Eigenschaften eines Strangs der Tabak verarbeitenden Industrie, insbesondere eines Tabakstrangs oder eines Filterstrangs, insbesondere einer Strangdichte, einer Feuchtigkeit, einer Beladung mit Zusatzstoffen, insbesondere mit Weichmacher oder Aktivkohle, zur Fremdkörpererkennung und/oder zur Erkennung von in einen Strang eingelegten Objekten, insbesondere flüssigkeitsgefüllten Kapseln.

## Claims

1. A microwave rod measurement device, in particular for measuring properties of a material rod (1) of the tobacco processing industry, comprising a microwave resonator housing (30) with at least one hollow cylindrical or rectangular-shaped interior (32, 52) configured as a microwave resonator (31, 51 - 51"'), through which a material rod (1) can pass, with end faces (31a, 31b, 51a, 51b) of the microwave resonator (31, 51 - 51"') having central feed-through openings for a material rod (1) in the symmetry axis (38, 58) of the microwave resonator (31, 51 - 51"'), further comprising two antennae (18, 18', 40, 45, 61, 65, 65'), which are arranged in the microwave resonator (31, 51 - 51'") for coupling a microwave measurement field into and out of the microwave resonator (31, 51 - 51"'), **characterized in that** at least one antenna passes completely through the microwave resonator (31, 51 - 51'") from one end face (31b, 51b) to the other end face (31a, 51a) and is connected in a conductive manner outside the microwave resonator (31, 51 - 51'") to a microwave generator and/or to an electronic measurement system on one side and is connected in a conductive manner to a ground on the opposite side.

2. The microwave rod measurement device according to claim 1, **characterized in that** both antennae (40, 45, 61, 65, 65') pass completely through the microwave resonator (31, 51 - 51"') from one end face (31b, 51b) to the other end face (31a, 51a).

3. The microwave rod measurement device according to claim 1 or 2, **characterized in that** the side or sides of the antenna (40, 45, 51, 65, 65') or antennae (40, 45, 51, 65, 65') connected to the ground is or are connected in a conductive manner at an end face (31a, 51a) of the microwave resonator (31, 51 - 51'") to the microwave resonator housing (30) such that the antenna (40, 45, 51, 65, 65') or the antennae (40, 45, 51, 65, 65') forms or form one or several inductive loop or loops with the microwave resonator housing (30).

4. The microwave rod measurement device according to any one of claims 1-3, **characterized in that** the antennae (18, 18', 40, 45, 61, 65, 65') are arranged essentially parallel to the longitudinal axis of the microwave resonator (31, 51 - 51'").

5. The microwave rod measurement device according to any one of claims 1-4, **characterized in that** at least one antenna (61, 65), which passes completely through the microwave resonator (51 - 51"') from one end face to the other end face, is broken, at least in sections, in its axial symmetry and is rotatably mounted in the microwave resonator (51 - 51"').

6. The microwave rod measurement device according to claim 5, **characterized in that** a rotatably mounted antenna (61, 65) has a thread on its grounded end, with which it can be screwed into the end face (31a, 51a) that forms a lid (33) of the microwave resonator housing (30), with a rotary element being arranged in particular on a ground-side end face of the antenna (61, 65).

7. The microwave rod measurement device according to claim 5 or 6, **characterized in that** a rotatably mounted antenna (61, 65) is inserted, at its end opposite the ground, through a dielectric junction (41, 46, 62, 66) in the end face (31b, 51b) that forms a bottom (34) of the microwave resonator (31, 51 - 51"') and has a connector that can be connected to a matching plug connection.

8. The microwave rod measurement device according to claims 1-7, **characterized in that** the antennae (40, 45, 61, 65, 65') are arranged in the microwave resonator (31, 51 - 51'") such that they couple into the field of the TM₀₁₀ mode of the microwave resonator (31, 51 - 51"') with minimum distortion.

9. A method of operating a microwave rod measurement device according to any one of claims 5-8, **characterized in that** a quality of the microwave resonator (31, 51 - 51'") is set by turning a rotatably mounted antenna (61, 65) in the microwave resonator, which passes completely through the microwave resonator (31, 51 - 51'") from one end face (31b, 51b) to the other end face (31a, 51a) and is broken, at least in sections, in its axial symmetry.

10. The method according to claim 9, **characterized in that** the turning takes place while the antenna (61, 65) is connected to a microwave generator or to an electronic measurement system.

11. Use of at least one microwave rod measurement device according to any one of claims 1-8 for detecting properties of a rod of the tobacco processing industry, in particular of a tobacco rod or of a filter rod, in particular of a rod density, a moisture content, a loading with additives, in particular with plasticizer or activated charcoal, for detecting foreign bodies and/or for detecting objects, in particular fluid-filled capsules, inserted in a rod.

## Revendications

1. Dispositif de mesure de boudin à micro-ondes, en particulier pour la mesure de propriétés d'un boudin de matériau (1) de l'industrie de transformation du tabac, comprenant
un boîtier de résonateur à micro-ondes (30) pourvu d'au moins un espace interne (32, 52) de forme cylindrique creuse ou cuboïde et conçu en tant que résonateur à micro-ondes (31, 51 - 51"'), lequel peut être traversé par un boudin de matériau (1), des surfaces d'extrémité (31a, 31b, 51a, 51b) du résonateur à micro-ondes (31, 51 - 51"') comportant, dans l'axe de symétrie (38, 58) du résonateur à micro-ondes (31, 51 - 51"'), des orifices de passage centraux d'un boudin de matériau (1),
deux antennes (18, 18', 40, 45, 61, 65, 65') qui sont disposées dans le résonateur à micro-ondes (31, 51 - 51"') pour le couplage et le découplage d'un champ de mesure de micro-ondes dans le résonateur à micro-ondes (31, 51 - 51"'),
**caractérisé en ce qu'**au moins une antenne traverse entièrement le résonateur à micro-ondes (31, 51 - 51"') d'une surface d'extrémité (31b, 51b) à l'autre surface d'extrémité (31a, 51a) et, à l'extérieur du résonateur à micro-ondes (31, 51 - 51"'), est reliée de manière conductrice sur un côté à un générateur de micro-ondes et/ou à une électronique de mesure, et est reliée de manière conductrice sur le côté opposé à une masse.

2. Dispositif de mesure de boudin à micro-ondes selon la revendication 1, **caractérisé en ce que** les deux antennes (40, 45, 61, 65, 65') traversent entièrement le résonateur à micro-ondes (31, 51 - 51"') d'une surface d'extrémité (31b, 51b) à l'autre surface d'extrémité (31a, 51a).

3. Dispositif de mesure de boudin à micro-ondes selon la revendication 1 ou 2, **caractérisé en ce que** le côté ou les côtés de l'antenne (40, 45, 51, 65, 65') ou des antennes (40, 45, 51, 65, 65') relié(s) à la masse est ou sont relié(s) de manière conductrice au boîtier de résonateur à micro-ondes (30), sur une surface d'extrémité (31a, 51a) du résonateur à micro-ondes (31, 51 - 51"'), de telle sorte que l'antenne (40, 45, 51, 65, 65') ou les antennes (40, 45, 51, 65, 65') forme(nt) avec le boîtier de résonateur à micro-ondes (30) une ou plusieurs boucles inductives.

4. Dispositif de mesure de boudin à micro-ondes selon l'une des revendications 1 à 3, **caractérisé en ce que** les antennes (18, 18', 40, 45, 61, 65, 65') sont disposées de manière sensiblement parallèle à l'axe longitudinal du résonateur à micro-ondes (31, 51 - 51"').

5. Dispositif de mesure de boudin à micro-ondes selon l'une des revendications 1 à 4, **caractérisé en ce qu'**au moins une antenne (61, 65) qui traverse entièrement le résonateur à micro-ondes (51 - 51'") d'une surface d'extrémité à l'autre surface d'extrémité est rompue au moins en partie dans sa symétrie axiale et est placée de façon à pivoter dans le résonateur à micro-ondes (51 - 51'").

6. Dispositif de mesure de boudin à micro-ondes selon la revendication 5, **caractérisé en ce qu'**une antenne (61, 65) placée de façon à pivoter présente à son extrémité reliée à la masse un filetage permettant de la visser dans la surface d'extrémité (31a, 51a) formant un couvercle (33) du boîtier de résonateur à micro-ondes (30), un élément de rotation étant disposé en particulier sur une surface d'extrémité côté masse de l'antenne (61, 65).

7. Dispositif de mesure de boudin à micro-ondes selon la revendication 5 ou 6, **caractérisé en ce qu'**une antenne (61, 65) placée de façon à pivoter est enfichée, à son extrémité opposée à la masse, par une transition diélectrique (41, 46, 62, 66), dans la surface d'extrémité (31b, 51b) formant un fond (34) du résonateur à micro-ondes (31, 51 - 51"') et présente un connecteur pouvant être raccordé à une connexion enfichable appropriée.

8. Dispositif de mesure de boudin à micro-ondes selon l'une des revendications 1 à 7, **caractérisé en ce que** les antennes (40, 45, 61, 65, 65') sont disposées dans le résonateur à micro-ondes (31, 51 - 51"') de façon à permettre leur couplage, le plus possible exempt de distorsions, dans le champ du mode TM₀₁₀ du résonateur à micro-ondes (31, 51 - 51"').

9. Procédé pour faire fonctionner un dispositif de mesure de boudin à micro-ondes selon l'une des revendications 5 à 8, **caractérisé en ce qu'**une qualité du résonateur à micro-ondes (31, 51 - 51"') est réglée par rotation d'une antenne (61, 65) placée de façon à pivoter dans le résonateur à micro-ondes, laquelle traverse entièrement le résonateur à micro-ondes (31, 51 - 51"') d'une surface d'extrémité (31b,51b)à l'autre surface d'extrémité (31a, 51a) et est rompue au moins en partie dans sa symétrie axiale.

10. Procédé selon la revendication 9, **caractérisé en ce que** la rotation s'effectue pendant que l'antenne (61, 65) est reliée à un générateur de micro-ondes ou à une électronique de mesure.

11. Utilisation d'au moins un dispositif de mesure de boudin à micro-ondes selon l'une des revendications 1 à 8 pour la détection de propriétés d'un boudin de l'industrie de transformation du tabac, en particulier d'un boudin de tabac ou d'un boudin de filtre, notamment d'une densité de boudins, d'une humidité, d'une charge en additifs, en particulier en plastifiants ou en charbons actifs, pour la détection de corps étrangers et/ou la détection d'objets insérés dans un boudin, en particulier de capsules remplies de liquide.
